# EUROPEAN PATENT APPLICATION

(11) **EP 3 978 124 A1**
(43) Date of publication of application: **06.04.2022**
(21) Application number: 21199554.3
(22) Date of filing: 28.09.2021
(51) Int. Cl.: B01L 3/00, G01N 1/40, G01N 27/447, B01L 7/00, C12Q 1/686, G01N 27/26, C12Q 1/68

(54) **NUCLEIC ACID DETECTION KIT AND NUCLEIC ACID DETECTION DEVICE**

(30) Priority: 30.09.2020 US 202063085368 P; 30.09.2020 US 202063085385 P; 31.05.2021 CN 202110602285
(71) Applicant: iCare Diagnostics International Co. Ltd., New Taipei 236 (TW); Century Technology (Shenzhen) Corporation Limited, Shenzhen, Guandong (CN)
(72) Inventor: LIN, YUAN-TIEN, 236 New Taipei (TW); LIU, MING-PANG, 236 New Taipei (TW); YEH, KUANG-CHEN, 236 New Taipei (TW); HSIEH, MING-YI, 236 New Taipei (TW); LEE, TAI-HSING, 236 New Taipei (TW); YANG, SHAO-FU, 236 New Taipei (TW); TUNG, LI-YU, 236 New Taipei (TW)
(74) Representative: Zaboliene, Reda

(57) **Abstract**

A nucleic acid detection kit (100, 200) includes a kit body (1), a detection chip (2), an electrophoresis box (3), and a connector (4). The detection chip (2) includes a channel (5) for carrying a microbead sample (a) of the acid. The detection chip (2) is connected to the electrophoresis box (3). The connector (4) is electrically connected to the detection chip (2) and the electrophoresis box (3). The microbead (a) undergoes a PCR amplification reaction to obtain a mixed microbead (b) in the channel. The mixed microbead (b) undergoes an electrophoretic detection in the electrophoresis box (3). A nucleic acid detection device (300) includes the nucleic acid detection kit (100, 200) is also disclosed. The nucleic acid detection device (300) has a simple structure, which is portable, flexible, and convenient, and can be used at home.

## Description

### FIELD

The subject matter relates to nucleic acid detection devices, and more particularly, to a nucleic acid detection kit and a nucleic acid detection device with the nucleic acid detection kit.

### BACKGROUND

Molecular diagnosis, morphological detection, and immunological detection are mostly carried out in laboratories. The detection process includes performing a PCR amplification reaction in a large and medium-sized detection equipment to acquire an amplified product. Then, the amplified product is manually transferred to an electrophoresis detection equipment for an electrophoretic detection. Finally, an electrophoretic detection result is manually transferred to a fluorescence analyzer to obtain a fluorescence image. However, such detection process is time-consuming, inefficient, and inflexible, and the detection device is not portable. The detection cannot be carried out anytime and anywhere.

### SUMMARY

To overcome the above shortcomings, the present disclosure provides a nucleic acid detection kit, including a kit body, a detection chip disposed in the kit body, an electrophoresis box disposed in the kit body, and a connector. The detection chip includes a first cover plate, a spacer layer, and a second cover plate, two opposite surfaces of the spacer layer are in contact with the first cover plate and the second cover plate, the first cover plate, the spacer layer, and the second cover plate cooperatively define a channel, the channel is configured to carry a microbead, the detection chip is connected to the electrophoresis box, the connector is electrically connected to the detection chip and the electrophoresis box, the microbead undergoes a PCR amplification reaction to obtain a mixed microbead in the channel, the mixed microbead undergoes an electrophoretic detection in the electrophoresis box.

In some embodiments, the detection chip further includes a driving circuit, a first dielectric layer, a conductive layer, and a second dielectric layer, the driving circuit is disposed on a surface of the first cover plate close to the second cover plate, the first dielectric layer is disposed on a side of the driving circuit close to the second cover plate, the conductive layer is disposed on a surface of the second cover plate close to the first cover plate, the second dielectric layer is disposed on a side of the conductive layer close to the first cover plate, the driving circuit and the conductive layer are respectively electrically connected to the connector, the first dielectric layer and the second dielectric layer cooperatively define the channel.

In some embodiments, the driving circuit includes a plurality of driving electrodes disposed in an array and a plurality of control electrodes, and the plurality of driving electrodes is electrically connected to the plurality of control electrodes.

In some embodiments, the detection chip farther includes a heating unit, the heating unit is disposed on a surface of the first cover plate away from the channel and / or the second cover plate away from the channel, and the heating unit is connected to the connector.

In some embodiments, the heating unit includes a heating layer and a heating circuit board respectively electrically connected to the heating layer.

In some embodiments, the heating circuit board includes a first circuit board, a second circuit hoard, and a connecting portion, the first circuit board is disposed on a surface of the first cover plate away from the channel, the second circuit board is disposed on a surface of the second cover plate away from the channel, the first circuit board and the second circuit board are electrically connected to each other through the connecting portion, and the first circuit board is inserted in the connector and electrically connected to the connector.

In some embodiments, the first circuit board and the second circuit board, and the connecting portion are an integrated structure.

In some embodiments, the driving circuit includes a sample adding area, a reagent storage area, a plurality of PCR amplification areas, and a solution outlet area, and the solution outlet area is connected to the electrophoresis box.

In some embodiments, the nucleic acid detection kit further includes a fluorescent reagent disposed in the reagent storage area.

In some embodiments, the electrophoresis box includes an electrophoretic body, two electrophoretic electrodes, a gel medium, and a capillary, the two electrophoretic electrodes are disposed on two ends of the electrophoresis body, the gel medium is disposed in the electrophoresis body, an end of the gel medium defines a liquid injection slot, each of the two electrophoresis electrodes is electrically connected to the connector, one end of the capillary enters the liquid injection slot, and the other end of the capillary enters the channel to connect to the detection chip.

In some embodiments, the electrophoresis box further includes two electrophoresis circuit boards, each of the two electrophoresis electrodes is electrically connected to one of the two electrophoresis circuit boards, each of the two electrophoretic circuit boards is electrically connected to the connector.

In some embodiments, one end of the capillary extends through the first cover plate into the channel defines a liquid inlet, the liquid inlet includes a plane or an inclined plane, the plane is parallel to an extension direction of the channel, and an angle is disposed between the inclined plane and a central axis of the capillary.

In some embodiments, the electrophoresis body is disposed on a side of the first cover plate away from the second cover plate, an opening of the electrophoresis body contacts the first cover plate.

In some embodiments, the electrophoresis box includes two electrophoretic electrodes, each of the two electrophoresis electrodes is electrically connected to the heating circuit board.

The present disclosure further provides a nucleic acid detection device, including the above nucleic acid detection kit and a nucleic acid detection host. A mounting groove is disposed on the nucleic acid detection host. The nucleic acid detection kit is detachably disposed in the mounting groove.

In some embodiments, the further including a host heating groove, a host sampling groove, and an image collection window, characterized in that the host heating groove is configured to receiving and heating the microbead, the host sampling groove is disposed above the mounting groove and connected to the mounting groove, the host sampling groove is configured to accommodate the microbead in the nucleic acid detection kit, the image collection window is disposed on a side of the mounting groove away from the host sampling groove, the image collection window corresponds to the electrophoresis box.

In some embodiments, relative to a top surface of the nucleic acid detection host, a height of an end of the mounting groove closed to the host sampling groove is higher than a height of another end of the mounting groove away from the host sampling groove.

The nucleic acid detection device provided by the present disclosure is integrated with the PCR amplification reaction and the electrophoresis detection of nucleic acid into in a single equipment through the cooperation of the nucleic acid detection host and the nucleic acid detection kit. Thus, the nucleic acid detection device has a simple structure, which is portable, flexible, and convenient, and can be used at home. At the same time, the detecting process is flexible, which does not need to be carried out in a professional laboratory.

### BRIEF DESCRIPTION OF THE DRAWINGS

Implementations of the present technology will now be described, by way of example only, with reference to the attached figures.
FIG. 1 is a front, top perspective view of an embodiment of a nucleic acid detection kit according to the present disclosure.
FIG. 2 is a rear, bottom perspective view of an embodiment of a nucleic acid detection kit according to the present disclosure.
FIG. 3 is an exploded diagrammatic view of an embodiment of a nucleic acid detection kit according to the present disclosure.
FIG. 4 is a rear, bottom perspective view of an embodiment of a nucleic acid detection kit without a kit body according to the present disclosure.
FIG. 5 is an exploded diagrammatic view of an embodiment of a nucleic acid detection kit without a kit body according to the present disclosure.
FIG. 6a is a diagrammatic view of an embodiment of a detection chip according to the present disclosure.
FIG. 6b is a cross-sectional view of an embodiment of a detection chip according to the present disclosure.
FIG. 7 is a diagrammatic view of an embodiment of a driving circuit of a detection chip according to the present disclosure.
FIG. 8 is a diagrammatic view of an embodiment of an electrophoresis box according to the present disclosure.

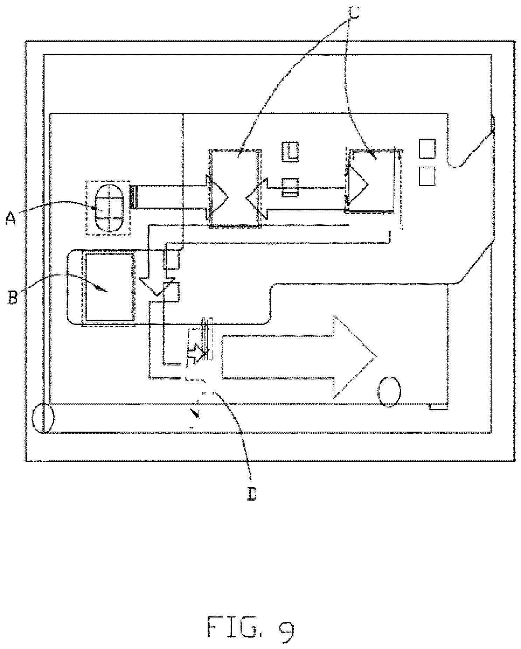

FIG. 9 is a diagrammatic view of an embodiment of a detection path of a sample in a nucleic acid detection kit according to the present disclosure.
FIG. 10 is a cross-sectional view of an embodiment of a detection chip and an electrophoresis box according to the present disclosure.
FIG. 11 is a cross-sectional view of another embodiment of a detection chip and an electrophoresis box according to the present disclosure.
FIG. 12 is a cross-sectional view of another embodiment of a detection chip and an electrophoresis box according to the present disclosure.
FIG. 13 is a diagrammatic view of another embodiment of a nucleic acid detection kit according to the present disclosure.
FIG. 14 is an exploded diagrammatic view of another embodiment of a nucleic acid detection kit according to the present disclosure.
FIG. 15 is a diagrammatic view of an embodiment of a nucleic acid detection device according to the present disclosure.
FIG. 16 is a diagrammatic view of an embodiment of a channel of a nucleic acid detection kit according to the present disclosure.

### DETAILED DESCRIPTION

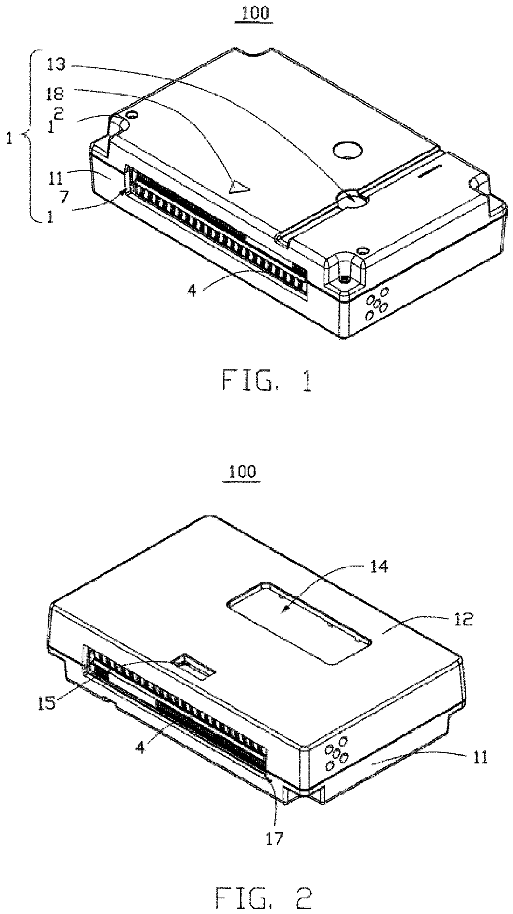

FIGS. 1, 3, 5, and 6b illustrate a nucleic acid detection kit 100, which includes a kit body 1, a detection chip 2, and an electrophoresis box 3, and a connector 4. The detection chip 2 is disposed in the kit body 1. The detection chip 2 and the electrophoresis box 3 are connected together and arranged in the kit body 1. Each of the detection chip 2 and the electrophoresis box 3 is electrically connected to the connector 4. The detection chip 2 is used to perform a PCR amplification reaction. The electrophoresis box 3 is used to perform an electrophoresis detection. The detection chip 2 includes a first cover plate 21, a spacer layer 22, and a second cover plate 23. Two opposite surfaces of the spacer layer 22 are in contact with the first cover plate 21 and the second cover plate 23 respectively. The first cover plate 21, the spacer layer 22, and the second cover plate 23 cooperatively define a channel 5. The channel 5 is configured to carry a solution to be detected. The solution in the channel 5 is in a form of microbead "a". The microbead "a" may undergo the PCR amplification reaction to obtain a mixed microbead "b", The mixed microbead "b" enters to the electrophoresis box 3 to undergo the electrophoresis detection. An image collection unit (not shown) acquires a fluorescent image within the electrophoresis box 3. The nucleic acid detection kit 100 integrates with the detection chip 2 and the electrophoresis box 3, which has a small size, and is suitable for a portable use. After the PCR amplification reaction is completed, the electrophoresis detection can be carried out automatically. The two processes are performed in a single equipment, and the sampling accuracy is precise. Thus, the detection process is efficient and flexible.

Referring to FIGS. 1 to 3, the kit body 1 includes a first housing 11, and a second housing 12. The second housing 12 defines a sampling port 13, and a detection window 14 is disposed on the first housing 11. The first housing 11 and the second housing 12 are connected together to define a receiving cavity (not shown in the figures). The detection chip 2, the electrophoresis box 3, and the connector 4 are disposed in the receiving cavity. The sampling port 13 is configured to correspond to the detection chip 2 such that the microbead "a" can be added into the detection chip 2 via the sampling port 13. The detection window 14 is configured to correspond to the electrophoresis box 3, so that the image collection unit can collect the fluorescent image of the electrophoresis box 3 through the detection window 14.

In an embodiment, the first housing 11 and the second housing 12 are assembled together by a latch or snapped together. The first housing 11 and the second housing 12 are further fastened together by screws to increase a connection strength therebetween.

In an embodiment, referring to FIGS. 1 to 3, an opening 17 is defined in a sidewall of the kit body 1. The opening 17 is configured to install the connector 4, which is electrically connected to an external power supply. The connector 4 is disposed in the receiving cavity, and exposed through the opening 17 to facilitate the electrical connection between the connector 4 and the external power supply.

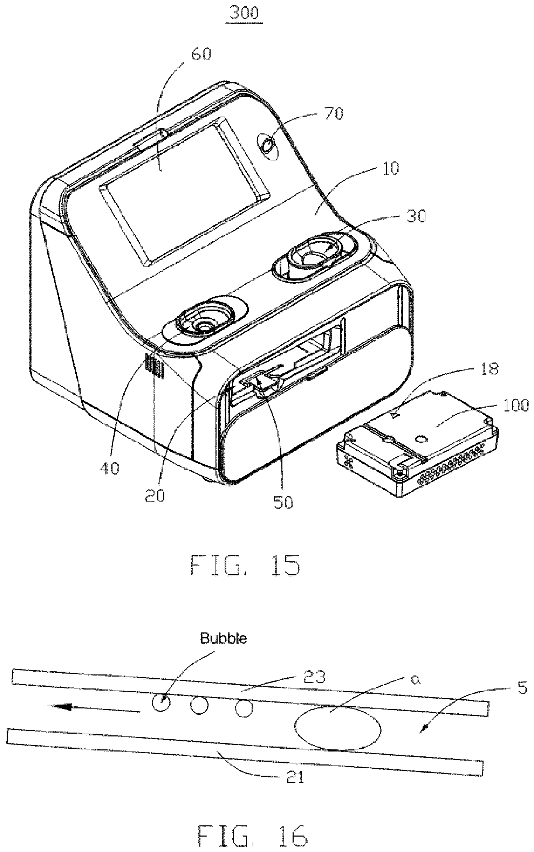

In an embodiment, referring to FIG. 2, the first housing 11 further defines a card slot 15. Referring to FIG. 15, a latching structure (not shown) on a mounting groove 20 of a nucleic acid detection device 300 is configured to enter the card slot 15 to clamp the nucleic acid detection kit 100 in place.

In an embodiment, referring to FIG. 1, an indication mark 18 (such as an arrow) is disposed on a side of the second housing 12 away from the receiving cavity. Referring to FIG. 15, the indication mark 18 is configured to indicate a direction of insertion of the nucleic acid detection kit 100 into the nucleic acid detection device 300 to avoid wrong insertion.

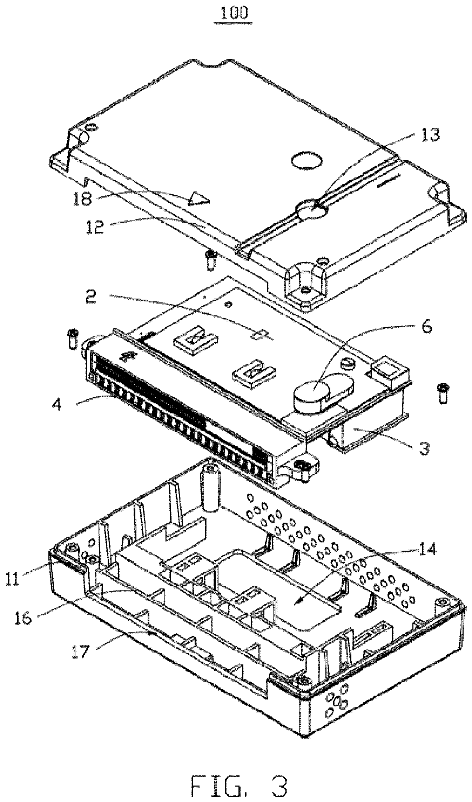

In an embodiment, referring to FIG. 3, several support structures 16 are disposed in the kit body 1. Because the detection chip 2, the electrophoresis box 3, and the connector 4 have different thickness, the support structures 16 with different heights are disposed to support the detection chip 2, the electrophoresis box 3, and the connector 4 in the kit body 1, achieving connection stability of the nucleic acid detection kit 100.

In an embodiment, the kit body 1 may be made of, but is not limited to, plastic.

In an embodiment, the support structures 16, the first housing 11, and the second housing 12 are integrally formed.

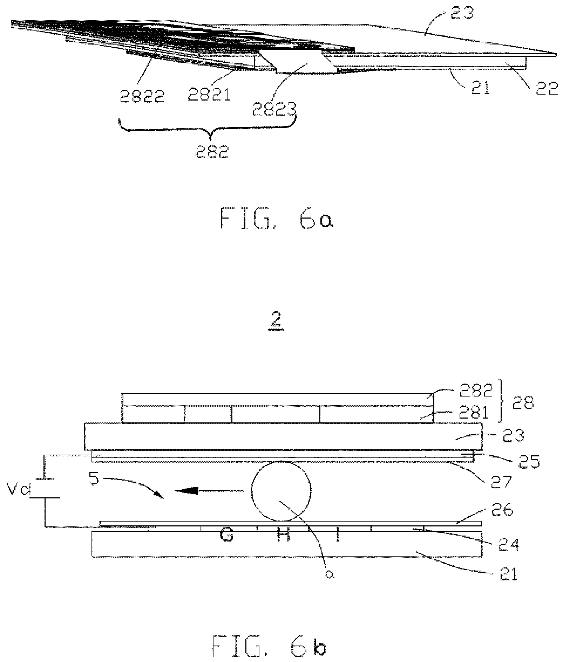

Referring to FIG. 6b, the detection chip 2 further includes a driving circuit 24, a first dielectric layer 26, a conductive layer 25, and a second dielectric layer 27. The driving circuit 24 is disposed on a surface of the first cover plate 21, and close to the second cover plate 23. The first dielectric layer 26 is disposed on a side of the driving circuit 24 close to the second cover plate 23. The conductive layer 25 is disposed on a surface of the second cover plate 23 close to the first cover plate 21. The second dielectric layer 27 disposed on a side of the conductive layer 25 close to the first cover plate 21. The driving circuit 24 and the conductive layer 25 are electrically connected to the connector 4. The microbead "a" can be driven to move along a flow path in the channel 5 by energizing or de-energizing a circuit between the driving circuit 24 and the conductive layer 25.

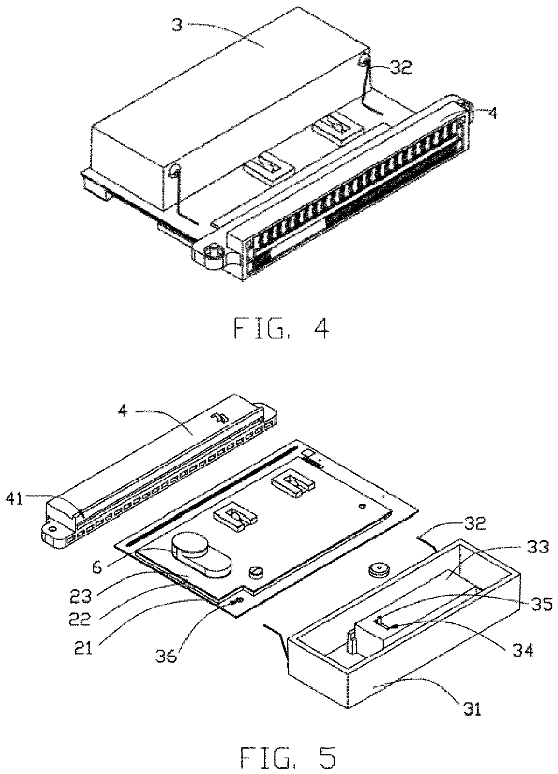

Referring to FIGS. 5, 6b, and 7, the driving circuit 24 includes a plurality of driving electrodes 241 disposed in an array and a plurality of control electrodes 242. Each of the driving electrodes 241 is electrically connected to one of the control electrodes 242. The control electrodes 242 are further electrically connected to the connector 4. In an embodiment, the driving circuit 24 is a thin film transistor (TFT) driving circuit. The microbead "a" has some conductivity, and can be driven by circuits between the driving electrodes 241 and the conductive layer 25 to move along the flow path in the channel 5 due to dielectric wetting principle (EWOD). Due to the EWOD principle, one of the circuits between one of the driving electrodes 241 and the conductive layer 25 can be selectively energized to change wetting characteristics between the microbead "a" and the first dielectric layer 26 and between the microbead "a" and the second dielectric layer 27, so as to control the microbead "a" to move along the flow path. Referring to FIG. 6b, the driving electrodes 241 include a driving electrode "1", a driving electrode "H", and a driving electrode "G". The microbead "a" moves on the driving electrode "I", the driving electrode "H", and the driving electrode "G". When the microbead "a" is on the driving electrode "H", a voltage "Vd" is applied between the driving electrode "G" and the conductive layer 25, and the driving electrode "H" is disconnected from the conductive layer 25. At this time, the wetting characteristics between the microbead "a" and the first dielectric layer 26, and between the microbead "a" and the second dielectric layer 27 are changed, so that a liquid-solid contact angle between the driving electrode "H" and microbead "a" becomes larger, and a liquid-solid contact angle between the driving electrode "G" and microbead "a" becomes smaller, to promote the movement of the microbead "a" from the driving electrode "H" to the driving electrode "G".

In an embodiment, the first dielectric layer 26 and the second dielectric layer 27 are insulated and are hydrophobic layers. On the one hand, the first dielectric layer 26 and the second dielectric layer 27 have the characteristics of insulation and hydrophobicity, and on the other hand, the first dielectric layer 26 and the second dielectric layer 27 can make the microbead "a" move smoothly along the flow path to avoid breakage or fragmentation of the microbead "a" during movement.

In an embodiment, each of the first dielectric layer 26 and the second dielectric layer 27 may be, but is not limited to, a polytetrafluoroethylene coating.

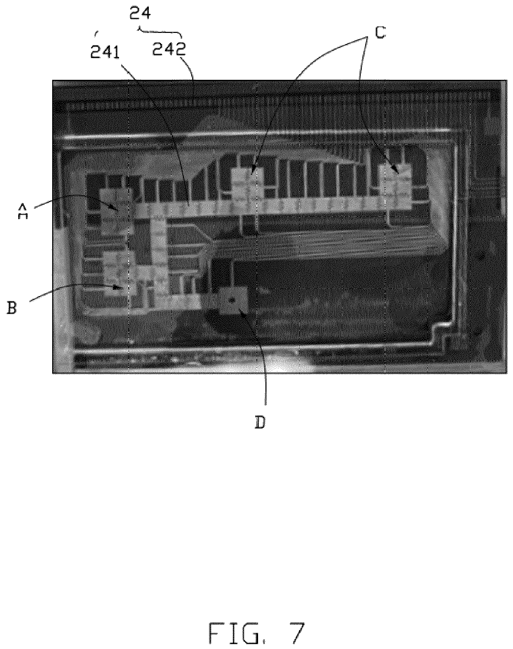

Referring to FIG. 7, in an embodiment, the driving circuit 24 may be formed on the surface of the first cover plate 21 by metal etching or electroplating.

In an embodiment, the control electrodes 242 are integrated at an edge of the first cover plate 21. An electrical connection between the detection chip 2 and the connector 4 is realized by inserting the side of the first cover plate 21 with the control electrodes 242 into the connector 4.

Referring to FIG. 7, in an embodiment, the driving circuit 24 can be divided into a plurality of areas according to their different purposes, including a sample adding area "A", a reagent storage area "B", a plurality of PCR amplification areas "C", and a solution outlet area "D". The detection chip 2 corresponding to the sample adding area "A" defines a detection kit sampling groove 6. The detection chip sampling groove 6 is configured to correspond to the sampling port 13 on the second cover plate 23. The microbead "a" is added in the sampling area "A" through the sampling port 13. The reagent storage area "B" is configured to store fluorescent reagents (such as fluorescent dyes or fluorescent probes). The microbead "a" undergoes PCR amplification reaction in the PCR amplification areas "C" to form an amplified product. The amplified product is mixed with a fluorescent reagent in the reagent storage area "B" to form a mixed microbead "b". The solution outlet area "D" is connected to the electrophoresis box 3. The mixed microbead "b" enters the electrophoresis box 3 through the solution outlet area "D". The number of the PCR amplification areas "C" can be determined according to an actual detection requirement.

After the microbead "a" enters the sampling area "A", the microbead "a" moves to the PCR amplification areas "C" and undergoes the PCR amplification reaction to form an amplified product. When the PCR amplification reaction is completed, the amplified product is moved to the reagent storage area "B" and mixed with the fluorescent reagent to obtain the mixed microbead "b". The mixed microbead "b" then enters the electrophoresis box 3 through the solution outlet area "D" and undergoes the electrophoretic detection.

In order to mix the amplified product and the fluorescent reagent more evenly, the mixed microbead "b" is moved back and forth several times in the PCR amplification area "C. A mixing area (not shown) can also be set separately in the driving circuit 24 to mix the amplified product and the fluorescent reagent.

In an embodiment, the number of the PCR amplification regions "C" is two, or three, or more.

In an embodiment, the fluorescent reagent (such as a fluorescent dye or a DNA probe) is within the reagent storage area "B" in advance. Thus, there is no need to add fluorescent reagent in the detection chip 2 separately.

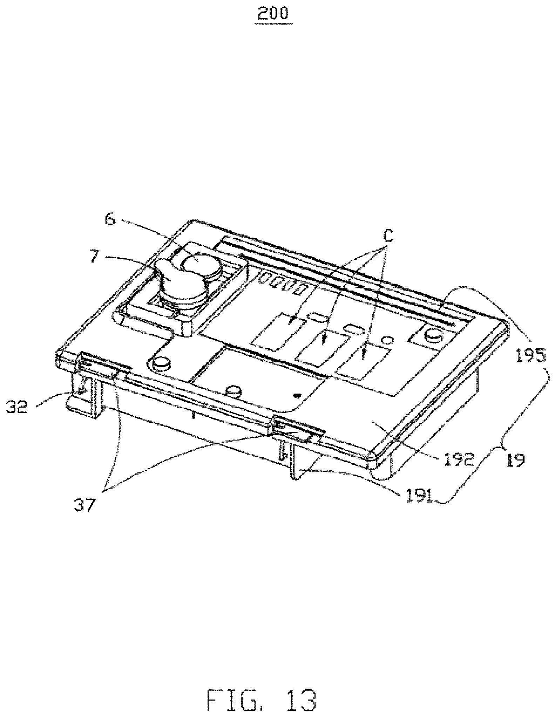

In yet another embodiment, referring to FIG. 13, the fluorescent reagent can also be separately added in the detection chip 2 to mix with the amplified product. The detection chip 2 defines a reagent tank 7 corresponding to the reagent storage area "B", and the fluorescent reagent can be added into the reagent tank 7 during the PCR amplification reaction. The type of the fluorescent reagent can be selected according to an actual need, which can improve the flexibility of the PCR amplification reaction.

Referring to FIGS. 3,5, and 6b, the detection chip 2 further includes a heating unit 28. The heating unit 28 is disposed on a surface of the first cover plate 21 away from the channel 5 and / or the second cover plate 23 away from the channel 5. The heating unit 28 corresponds to the PCR amplification regions "C" and is connected to the connector 4. The heating unit 28 heats the microbead "a" to undergo the PCR amplification reaction.

In an embodiment, the heating unit 28 includes a heating layer 281 and a heating circuit board 282 electrically connected to the heating layer 281. The heating circuit board 282 is further electrically connected to the connector 4. The heating layer 281 is energized through the heating circuit board 282 to heat some heatable areas of the channel 5.

In an embodiment, the heatable areas of the channel 5 includes the PCR amplification regions "C" and the reagent storage area "B".

In an embodiment, the heating layer 281 includes a carbon nanotube heating layer. The heating layer 281 uniformly heats the heatable areas due to a uniformity heat conduction in a horizontal direction of the carbon nanotube heating layer. At the same time, the heating layer 281 can avoid violent temperature changes during heating. The heating layer 281 also allows the heatable areas to have a lower heat loss and a higher heating efficiency.

In yet another embodiment, the heating layer 281 may be made of, but is not limited to, metal and graphite.

In an embodiment, the heating unit 28 is disposed on the surface of the second cover plate 23 away from the channel 5.

In an embodiment, the heating unit 28 is disposed on the surface of the second cover plate 23 away from the channel 5 through a thermally conductive adhesive layer (not shown).

In an embodiment, the heating circuit board 282 includes a circuit (not shown) corresponding to the PCR amplification areas "C" and the reagent storage area "B". After the heating circuit board 282 is powered on, the circuit on the heating circuit board 282 can heat the PCR amplification areas "C" and the reagent storage area "B" to certain precise temperatures, and each temperature of the PCR amplification areas "C" and the reagent storage area "B" is easy to control.

In an embodiment, the heating layer 281 includes two heatable areas. Each of the two heatable areas corresponds to a PCR amplification area "C". One of the two heatable areas has a temperature range from 90 °C to 105 °C. The other one of has a temperature range from 40 °C to 75 °C.

In yet another embodiment, the heating layer 281 includes three heatable areas. Each of the three heatable areas corresponds to a PCR amplification area "C". One of the three heatable areas has a temperature range from 90 °C to 105 °C. A second one of the three heatable areas has a temperature range from 68 °C to 75 °C. A third one of the three heatable areas has a temperature range from 40 °C to 65 °C.

In an embodiment, referring to FIGS. 4, 5, 6a, and 6b, the heating circuit board 282 includes a first circuit board 2821, a second circuit board 2822, and a connecting portion 2823. The first circuit board 2821 is disposed on a surface of the first cover plate 21 away from the channel 5. The second circuit board 2822 is disposed on a surface of the second cover plate 23 away from the channel 5. The first circuit board 2821 and the second circuit board 2822 arc electrically connected to each other through the connecting portion 2823. The first circuit board 2821 is inserted in a slot 41 of the connector 4 to realize an electrical connection between the heating unit 28 and the connector 4. The first circuit board 2821 and the second circuit board 2822 can together heat the microbead "a" in the channel 5 more evenly. In addition, the electrical connection of the first circuit board 2821 and the second circuit board 2822 is realized through the connecting portion 2823, achieving convenient assembly of the heating unit 28 in the detection chip 2. Furthermore, output wirings are only found on the first circuit board 2821, which is convenient to connect to the connector 4.

In an embodiment, the first circuit board 2821, the second circuit board 2822, and the connecting portion 2823 are integrally formed.

In an embodiment, silicone oil may be injected into the channel 5 after the detection chip 2 is assembled, and the microbead "a" is driven to move in the silicone oil.

Referring to FIG. 2, in an embodiment, the first cover plate 21 and the second cover plate 23 are glass plates. The spacer layer 22 is a double-sided adhesive frame, which is connected to edges of the first cover plate 21 and the second cover plate 23 to cooperatively define the channel 5. A volume of the channel 5 can be adjusted by changing a thickness of the spacer layer 22 according to an actual demand.

Referring to FIG. 3 to 5, and 8, the electrophoresis box 3 includes an electrophoretic body 31, two electrophoretic electrodes 32, a gel medium 33, and a liquid injection slot 34. Two electrophoretic electrodes 32 are disposed on two ends of the electrophoresis body 31. The electrophoretic body 31 defines an electrophoretic groove 314, the gel medium 33 is disposed in the electrophoresis groove 314. The liquid injection slot 34 is disposed on an end of the gel medium 33. The electrophoresis box 3 further includes a capillary 35. The capillary 35 is disposed at one end of the gel medium 33. Each of the two electrophoresis electrodes 32 is electrically connected to the connector 4. One end of the capillary 35 enters in the liquid injection slot 34, and the other end of the capillary 35 enters the channel 5 to connect to the detection chip 2. The mixed microbead "b" on the solution outlet area "D" may enter the liquid injection slot 34 of the gel medium 33 through the capillary 35, thereby presenting itself for the electrophoresis detection.

Referring to FIGS. 3, 5, and 8, the electrophoresis body 31 is disposed on a side of the first cover plate 21 away from the second cover plate 23. An opening of the electrophoresis body 31 faces the first cover plate 21. The electrophoresis body 31 includes a transparent substrate 311 and a plurality of sidewalk 312 connected to the transparent substrate 311. The transparent substrate 311 and the sidewalls 312 cooperatively form the electrophoretic groove 314. Ends of the sidewalls 312 away from the transparent substrate 311 are connected to the surface of the first cover plate 21, thereby, the electrophoresis body 31 is covered by the first cover plate 21. With the above configuration, the electrophoresis box 3 can better connect to the detection chip 2, which facilitates the transfer of the mixed microbead "b" from the detection chip 2 to the electrophoresis box 3. The nucleic acid detection kit 100 is integrated with the detection chip 2 and the electrophoresis box 3, which has a small size, and is suitable for the nucleic acid detection device 100.

In an embodiment, a sealing rubber ring (not shown) is disposed between the sidewall 312 and the first cover plate 21 to improve sealing of the electrophoresis box 3.

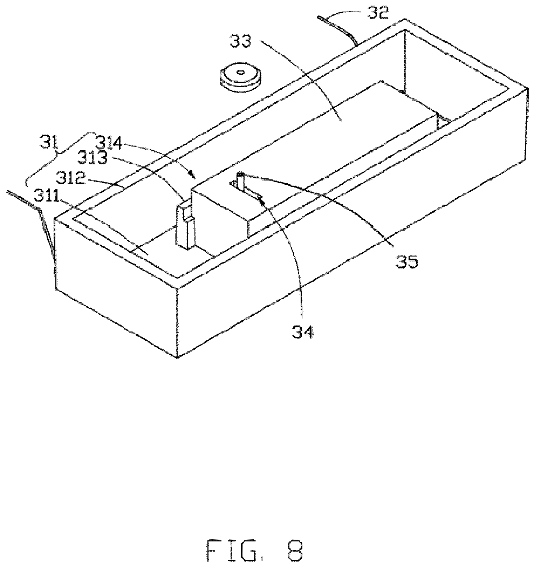

Referring to FIG. 8, the electrophoresis body 31 further includes a plurality of latching portions 313 disposed on the transparent substrate 311. The latching portions 313 fix the gel medium 33, and prevent the gel medium 33 from moving out of position, thereby guaranteeing the accuracy of the electrophoresis detection.

In an embodiment, the gel medium 33 is substantially cubic.

In an embodiment, the transparent substrate 311 is a transparent glass plate, and the fluorescence image of the electrophoresis box 3 can be observed on a side of the transparent substrate 311 away from the gel medium 33.

In an embodiment, there are four latching portions 313. Four clamping portions 313 are disposed outside of the gel medium 33 to fix the gel medium 33.

In an embodiment, a liquid injection hole 36 is disposed in the first cover plate 21. The liquid injection hole 36 corresponds to the electrophoresis box 3. A buffer can be injected into the electrophoresis groove 314 through the liquid injection hole 36.

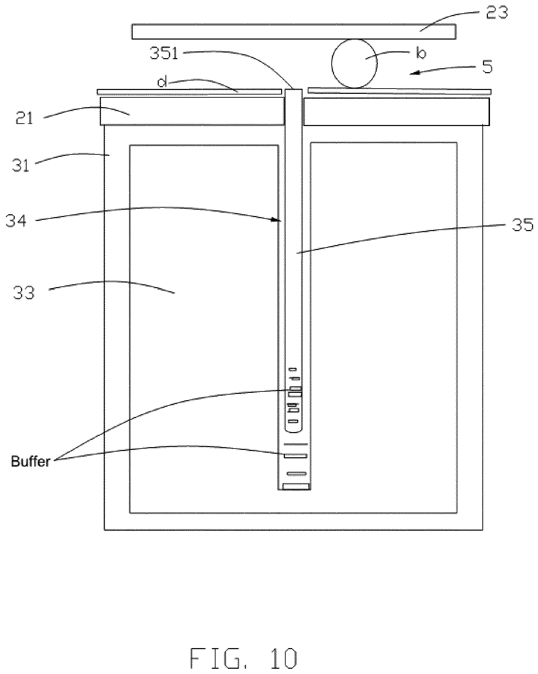

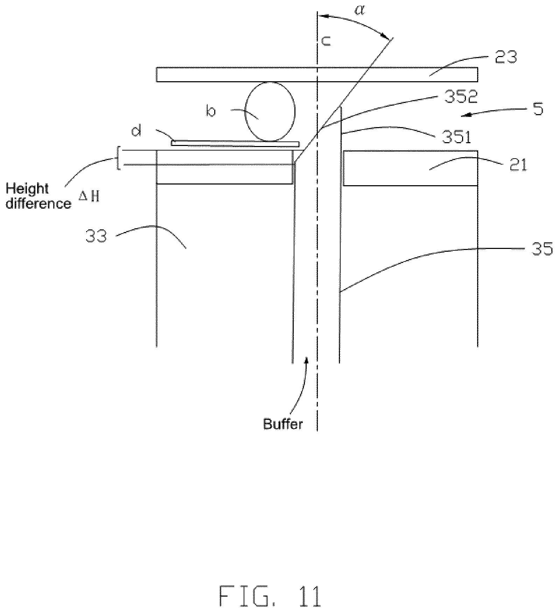

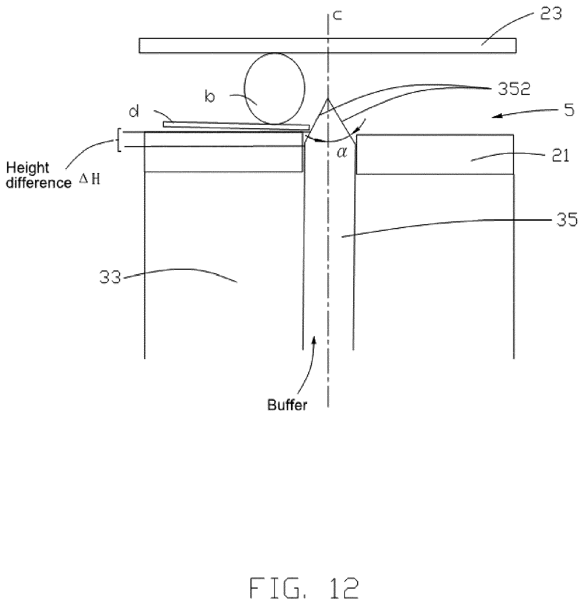

Referring to FIGS. 10 to 12, and 15, one end of the capillary 35 passes through the first cover plate 21 into the channel 5. The capillary 35 includes a liquid inlet 351, which is located in the channel 5. The mixed microbead "b" enters the gel medium 33 through the capillary 35. Referring to FIG. 10, for smooth entry of the mixed microbead "b" into the electrophoresis box 3, an end of the liquid inlet 351 needs to be flush with the liquid level of silicone oil "d". Alternatively, referring to FIGS. 11 and 12, the liquid inlet 351 includes at least one inclined plane 352. The lowest point of the inclined plane 352 is lower than the bottom surface of the channel 5. There is a height difference "Δ H" between the lowest point of the inclined plane 352 and the bottom surface of the channel 5. A liquid level of the silicone oil "d" is connected to the inclined plane 352, which makes the mixed microbead "b" smoothly enter the capillary 35. During the assembly of the capillary 35 and the detection chip 2, the capillary 35 needs to be filled with buffer, and the buffer in the capillary 35 needs to contact the mixed mierobead "b" in the solution outlet area "D" to form a continuous flow and ensure the mixed microbead "b" enters the electrophoresis box 3 smoothly.

In an embodiment, an angle between the inclined plane 352 and a central axis "C" of the capillary 35 ranges from 45 degrees to 60 degrees. The inclined plane 352 being in this angle ensures smooth entry of the mixed microbead "b" into the electrophoresis box 3.

In yet another embodiment, referring to FIG. 12, liquid inlet 351 incudes two inclined planes 352. An angle between each of the inclined planes 352 and the central axis "C" of the capillary 35 ranges from 45 degrees to 60 degrees.

Referring to FIG. 4, one end of each electrophoresis electrodes 32 extends into the electrophoresis body 31, and the other end of is electrically connected to the heating circuit board 282 of the heating unit 28. The electrophoresis electrodes 32 are directly connected to the heating circuit board 282 of the heating unit 28 to avoid additional and complex circuitry between the electrophoresis electrodes 32 and the connector 4.

Referring to FIG. 8, an assembly process of the electrophoresis box 3 includes the following steps.

At step one, an electrophoresis electrode 32 is installed on each end of the electrophoresis body 31. One end of each electrophoresis electrode 32 extends into the electrophoresis body 31, and the other end is electrically connected to the heating circuit board 282 of the heating unit 28.

At step two, the gel medium 33 is disposed in the electrophoresis groove 314 of the electrophoresis body 31 and fixed among the four latching portions 313. The liquid injection slot 34 is disposed on an end of the gel medium 33, and the opening of the liquid injection slot 34 faces the detection chip 2.

At step three, the buffer is injected into the electrophoresis body 31.

At step four, a glue layer is disposed on the end of the sidewall 312.

At step five, the first cover plate 21 is applied on the glue layer to cover the electrophoresis body 31.

At step six, the buffer is injected into the electrophoresis body 31 through the injection hole 36.

At step seven, the liquid injection hole 36 is covered by a breathable film or a release film.

A method for using the nucleic acid detection kit 20 to perform the PCR amplification reaction and the electrophoresis detection includes followings steps.

At step one, referring to FIG. 3, a solution to be detected is injected into the detection kit sampling groove 6 through the sampling port 13. A nucleic acid sample is within the solution.

At step two, referring to FIG. 15, the solution in the detection kit sampling groove 6 is adding into the detection chip 2 in the form of microbead "a" by pressure control.

At step three, referring to FIG. 15, the microbead "a" is driven by circuits between the driving electrodes 241 and the conductive layer 25 to move on the flow path in the channel 5 to perform the PCR amplification reaction to form an amplified product.

There are two PCR amplification areas "C". One of the two PCR amplification areas "C" has a temperature range from 90 °C to 105 °C, and the other one has a temperature range from 40 °C to 75 °C.

In an embodiment, the PCR amplification reaction includes the following four steps. At step one, a thermal denaturation of the microbead "a" is performed at a temperature range from 90 °C to 105 °C for 15 min to 25 min. At step two, a RT reverse transcription of the microbead "a" after the thermal denaturation is performed at a temperature range from 45 °C to 60 °C for 5 min to 15 min. At step three, the microbead "a" after the RT reverse transcription is heated at a temperature range from 90 °C to 100 °C for 1 min to 5 min. At step four, the microbead "a" is heated at a temperature range from 90 °C to 100 °C for 20 seconds to 50 seconds, then heated at a temperature range from 55 °C to 65 °C for 40 seconds to 60 seconds. The fourth step is repeated in a range from 35 cycles to 50 cycles (such as 40 cycles) to form an amplified product. In an embodiment, a temperature sensor and a time relay are used to sense the temperature and the heating time.

In yet another embodiment, the PCR amplification reaction includes the following four steps. At step one, a thermal denaturation of the microbead "a" is performed at a temperature range from 90 °C to 105 °C for 3 min to 8 min. At step two, an amplification reaction of the microbead "a" after the thermal denaturation is performed at a temperature range from 45 °C to 60 °C for 3 min to 8 min. At step three, the microbead "a" after the amplification reaction is heated at a temperature range from 90 °C to 100 °C for 3 min to 8 min. At step four, the microbead "a" is heated at a temperature range from 90 °C to 100 °C for 3 seconds to 8 seconds, then heated at a temperature range from 50 °C to 65 °C for 10 seconds to 20 seconds, then heated at a temperature range from 68 °C to 75 °C for 10 seconds to 20 seconds. The fourth step is repeated in a range from 35 cycles to 50 cycles to form an amplified product.

In an embodiment, at step one, a thermal denaturation of the microbead "a" is performed at a temperature range from 90 °C to 97 °C for 3 min to 5 min. At step two, an amplification reaction of the microbead "a" after the thermal denaturation is performed at a temperature range from 55 °C to 60 °C for 3 min to 5 min. At step three, the microbead "a" after the amplification reaction is heated at a temperature range from 95 °C to 97 °C for 3 min to 8 min. At step four, the microbead "a" is heated at a temperature range from 95 °C to 97 °C for 3 seconds to 5 seconds, then heated at a temperature range from 55 °C to 60 °C for 15 seconds to 20 seconds, then heated at a temperature range from 70 °C to 72 °C for 15 seconds to 20 seconds. The fourth step is repeated in a range from 43 cycles to 45 cycles (such as 45 cycles) to form an amplified product.

At step four, referring to FIG. 3, the amplified product is mixed with the fluorescent reagent preplaced in the reagent storage area "B" to form the mixed microbead "b", and the mixed microbead "b" is driven to enter the electrophoresis box 3.

At step five, the electrophoresis box 3 is controlled to perform the electrophoretic detection.

In an embodiment, the nucleic acid detection kit 100 is substantially cubic.

In an embodiment, the nucleic acid detection kit 100 is disposable. The nucleic acid detection kit 100 has no need to be cleaned after used.

The nucleic acid detection kit 100 provided by the present disclosure is integrated with the PCR amplification reaction and the electrophoresis detection into in a single equipment. The connection of the PCR amplification reaction and the electrophoresis detection is smoothly, which greatly improves the detection efficiency. Thus, the nucleic acid detection kit 100 has a simple structure, which is portable, flexible, and convenient, and can be used at home. At the same time, the detecting process is flexible, which does not need to be carried out in a professional laboratory.

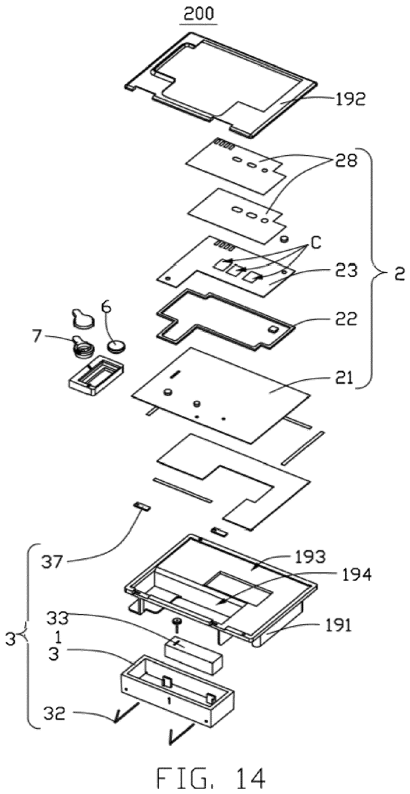

FIG. 13 to 14 illustrate another nucleic acid detection kit 200, which further includes a mounting bracket 19 in the kit body 1. The mounting bracket 19 is configured to improve the connection stability of the detection chip 2, the electrophoresis box 3, and the connector 4 in the kit body 1. The detection chip 2, the electrophoresis box 3, and the connector 4 are installed and fixed on the mounting bracket 19.

The mounting bracket 19 includes a bracket body 191 and a bracket cover plate 192. The bracket body 191 includes a detection chip installation area 193 and an electrophoresis box installation area 194. The detection chip 2 is installed and fixed in the detection chip installation area 193, and the electrophoresis box 3 is installed in the electrophoresis box installation area 194.

The bracket cover plate 192 includes a window 195 corresponding to the detection chip 2. The detection chip 2 is exposed through the window 195 to facilitate the electrical connection between the detection chip 2 and another connector (not shown). In an embodiment, the connector can be disposed above the detection chip 2.

In an embodiment, the support cover plate 192 and the frame body 191 may be bonded and fixed by double-sided adhesive.

In an embodiment, the electrophoresis box 3 in the nucleic acid detection kit 200 further includes two electrophoresis circuit boards 37. One end of each electrophoresis electrodes 32 extends into the electrophoresis body 31, and the other end is electrically connected to one electrophoresis circuit board 37. The electrophoretic circuit boards 37 are electrically connected to the connector (not shown). The two electrophoresis circuit boards 37 correspond to the electrophoresis electrodes 32.

FIG. 15 illustrates a nucleic acid detection device 300 according to the present disclosure. The nucleic acid detection device 300 includes a nucleic acid detection host 10, and the nucleic acid detection kit 100 (or 200). The mounting groove 20 is disposed on the nucleic acid detection host 10. The nucleic acid detection kit 100 (or 200) is detachably disposed in the mounting groove 20.

Referring to FIG. 15, the nucleic acid detection device 300 further includes a host heating groove 30, a host sampling groove 40, and an image collection window 50. The host heating groove 30 is configured to receiving and heating the microbead "a". The host sampling groove 40 is disposed above the mounting groove 20 and connected to the mounting groove 20. The host sampling groove 40 is configured to add the microbead "a" into the nucleic acid detection kit 100 (or 200). The image collection window 50 is disposed on a side of the mounting groove 20 away from the host sampling groove 40. An image collection unit (not shown) is disposed on a side of the image collection window 50 away from the mounting groove 20. The image collection unit is configured to collect the fluorescence image within the electrophoresis box 3 through the image collection window 50 and the detection window 14 on the nucleic acid detection kit 100 (or 200).

In an embodiment, referring to FIG. 15 and 16, the mounting groove 20 is inclined relative to a top surface of the nucleic acid detection host 10, which can make the nucleic acid detection kit 100 (or 200) be placed obliquely in the mounting groove 20. In an embodiment, relative to the top surface of the nucleic acid detection host 10, a height of an end of the mounting groove 20 closed to the host sampling groove 40 is higher than a height of another end of the mounting groove 20 away from the host sampling groove 40. Bubbles will be generated during a PCR amplification reaction in the nucleic acid detection kit 100 (or 200). The bubbles may stay in and block a flow path of microbead "a" in the nucleic acid detection kit 100 (or 200), so that the microbead "a" cannot move along the flow path to cause a failure of the nucleic acid detection. Therefore, the mounting groove 20 is designed to be inclined, so that the nucleic acid detection kit 100 (or 200) can be placed obliquely, and the bubbles generated by the PCR amplification reaction can be discharged out without hindering the movement of the microbead "a".

In an embodiment, the nucleic acid detection device 300 further includes a display screen 60 and a camera 70. The display screen 60 is configured to display an operation interface to allow a user to set operation parameters, and disposed to display the fluorescent image. The camera 70 is configured to record an operation process of the user, and collect relevant information of the detection solution (such as information indicating a source of the nucleic acid sample).

Referring to FIG. 15, a method for detecting the nucleic acid through the nucleic acid detection device 300 including the flowing steps.

At step one, operation parameters are set in the nucleic acid detection device 300. The nucleic acid detection host 10 is turned on and the operation parameters are set in the nucleic acid detection host 10 through the display screen 60.

In an embodiment, the operation parameters include the temperature and the heating time of the host heating groove 30, process parameters of the PCR amplification reaction, and process parameters of the electrophoresis detection.

At step two, the nucleic acid detection kit 100 is inserted into the mounting groove 20.

At step three, the nucleic acid sample is collected and mixed with a detection reagent to form a solution to be detected. The solution is then heated in the host heating groove 30.

At step four, the detection solution is transferred from the host heating groove 30 into the nucleic acid detection kit 100 to undergo the PCR amplification reaction and the electrophoresis detection.

In an embodiment, the solution is quantitatively sucked 10 - 30 µl (preferably 20 µl) from the host heating groove 30 and added into the nucleic acid detection kit 100. The solution containing the nucleic acid sample is in the form of microbead "a" in the channel 5. The microbead "a" undergoes the PCR amplification reaction in the detection chip 2. After amplification, the amplified product is combined with the fluorescent reagent within the detection chip 2 to form a mixed microbead "b". Then the mixed microbead "b" is driven to enter the electrophoresis box 3 from the detection chip 2 to undergo the electrophoresis detection.

At step five, an electrophoretic detection result (such as the fluorescent image) is acquired by the image collection unit.

After the electrophoretic detection, the fluorescent image is acquired by the image collection unit through the image collection window 50 and the detecting window 14. The fluorescent image is processed by the image processor, and then displayed on the display screen 60. The fluorescent image can also be uploaded and sent to the client for the user to consult.

With the above configuration, the nucleic acid detection device 300 provided by the present disclosure is integrated with the PCR amplification reaction and the electrophoresis detection of nucleic acid into in a single equipment through the cooperation of the nucleic acid detection host 10 and the nucleic acid detection kit 100 (or 200). Thus, the nucleic acid detection device 100 (or 200) has a simple structure, which is portable, flexible, and convenient, and can be used at home. At the same time, the detecting process is flexible, which does not need to be carried out in a professional laboratory.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts within the principles of the present disclosure, up to and including, the fall extent established by the broad general meaning of the terms used in the claims.

## Claims

1. A nucleic acid detection kit (100, 200), comprising a kit body (1), **characterized in that**, the nucleic acid detection kit (100, 200) further comprising:
a detection chip (2) disposed in the kit body (1);
an electrophoresis box (3) disposed in the kit body (1); and
a connector (4);
the detection chip (2) comprises a first cover plate (21), a spacer layer (22), and a second cover plate (23), two opposite surfaces of the spacer layer (22) are in contact with the first cover plate (21) and the second cover plate (23), the first cover plate (21), the spacer layer (22), and the second cover plate (23) cooperatively define a channel (5), the channel (5) is configured to carry a microbead (a), the detection chip (2) is connected to the electrophoresis box (3), the connector (4) is electrically connected to the detection chip (2) and the electrophoresis box (3), the microbead (a) undergoes a PCR amplification reaction to obtain a mixed microbead (b) in the channel (5), the mixed microbead (b) undergoes an electrophoretic detection in the electrophoresis box (3).

2. The nucleic acid detection kit (100) of claim 1, **characterized in that**, the detection chip (2) further comprises a driving circuit (24), a first dielectric layer (26), a conductive layer (25), and a second dielectric layer (27), the driving circuit (24) is disposed on a surface of the first cover plate (21) close to the second cover plate (23), the first dielectric layer (21) is disposed on a side of the driving circuit (24) close to the second cover plate (23), the conductive layer (25) is disposed on a surface of the second cover plate (23) close to the first cover plate (21), the second dielectric layer (27) is disposed on a side of the conductive layer (25) close to the first cover plate (21), the driving circuit (24) and the conductive layer (25) are respectively electrically connected to the connector (4), the first dielectric layer (26) and the second dielectric layer (27) cooperatively define the channel (5).

3. The nucleic acid detection kit (100) of claim 2, **characterized in that**, the driving circuit (24) comprises a plurality of driving electrodes (241) disposed in an array and a plurality of control electrodes (242), and the plurality of driving electrodes (241) is electrically connected to the plurality of control electrodes (242).

4. The nucleic acid detection kit (100) of claim 1, **characterized in that**, the detection chip (2) further comprises a heating unit (28), the heating unit (28) is disposed on a surface of the first cover plate (21) away from the channel (5) and/or on a surface of the second cover plate (23) away from the channel (5), and the heating unit (28) is connected to the connector (4).

5. The nucleic acid detection kit (100) of claim 4, **characterized in that**, the heating unit (28) comprises a heating layer (281) and a heating circuit board (282) each electrically connected to the heating layer (281).

6. The nucleic acid detection kit (100) of claim 5, **characterized in that**, the heating circuit board (282) comprises a first circuit board (2821), a second circuit board (2822), and a connecting portion (2823), the first circuit board (2821) is disposed on a surface of the first cover plate (21) away from the channel (5), the second circuit board (2822) is disposed on a surface of the second cover plate (23) away from the channel (5), the first circuit board (2821) and the second circuit board (2822) are electrically connected to each other through the connecting portion (2823), and the first circuit board (2821) is inserted in the connector (4) and electrically connected to the connector (4).

7. The nucleic acid detection kit (100) of claim 6, **characterized in that**, the first circuit board (2821), the second circuit board (2822), and the connecting portion (2823) are an integrated structure.

8. The nucleic acid detection kit (100) of claim 2, **characterized in that**, the driving circuit comprises a sample adding area (A), a reagent storage area (B), a plurality of PCR amplification areas (C), and a solution outlet area (S), and the solution outlet area (D) is connected to the electrophoresis box (3).

9. The nucleic acid detection kit (100) of claim 8, **characterized in that**, the nucleic acid detection kit (100) further comprises a fluorescent reagent disposed in the reagent storage area (B).

10. The nucleic acid detection kit (100) of claim 1, **characterized in that**, the electrophoresis box (3) comprises an electrophoretic body (31), two electrophoretic electrodes (32), a gel medium (33), and a capillary (35), the two electrophoretic electrodes (32) are disposed on two ends of the electrophoresis body (31), the gel medium (33) is disposed in the electrophoresis body (31), an end of the gel medium (33) defines a liquid injection slot (34), each of the two electrophoresis electrodes (32) is electrically connected to the connector (4), one end of the capillary (35) enters the liquid injection slot (34), and the other end of the capillary (35) enters the channel (5) to connect to the detection chip (2).

11. The nucleic acid detection kit (200) of claim 10, **characterized in that**, the electrophoresis box (3) further comprises two electrophoresis circuit boards (37), each of the two electrophoresis electrodes (32) is electrically connected to one of the two electrophoresis circuit boards (37), each of the two electrophoretic circuit boards (37) is electrically connected to the connector.

12. The nucleic acid detection kit (100) of claim 10, **characterized in that**, one end of the capillary (35) extends through the first cover plate (21) into the channel (5) and defines a liquid inlet (351), the liquid inlet (351) comprises a plane or an inclined plane (352), the plane is parallel to an extension direction of the channel (5), and an angle is disposed between the inclined plane (352) and a central axis of the capillary (35).

13. The nucleic acid detection kit (100) of claim 10, **characterized in that**, the electrophoresis body (31) is disposed on a side of the first cover plate (21) away from the second cover plate (23), an opening of the electrophoresis body (31) contacts the first cover plate (21).

14. The nucleic acid detection kit (100) of claim 5, **characterized in that**, the electrophoresis box (31) comprises two electrophoretic electrodes (32), each of the two electrophoresis electrodes (32) is electrically connected to the heating circuit board (282).

15. A nucleic acid detection device (300), **characterized in that**, comprising:
a nucleic acid detection kit (100, 200) of any one of claims 1 to 14; and
a nucleic acid detection host (10), a mounting groove (20) disposed on the nucleic acid detection host (10), the nucleic acid detection kit (100, 200) detachably disposed in the mounting groove (20).
